(19) Europäisches Patentamt
European Patent Office
Office européen des brevets

(11) Veröffentlichungsnummer: **0 386 715 B1**

# (12) EUROPÄISCHE PATENTSCHRIFT

(45) Veröffentlichungstag der Patentschrift: **15.02.95**

(21) Anmeldenummer: **90104312.5**

(22) Anmeldetag: **07.03.90**

Die Akte enthält technische Angaben, die nach dem Eingang der Anmeldung eingereicht wurden und die nicht in dieser Patentschrift enthalten sind.

(51) Int. Cl.$^6$: **C07D 213/64**, A01N 43/40, A01N 43/10, A01N 43/08, A01N 43/54, A01N 43/78, C07D 333/12, C07D 307/12, C07D 239/26, C07D 277/22, C07D 213/643

## (54) Heteroaryl-aryl-buten- und -butanderivate, Verfahren zu ihrer Herstellung, sie enthaltende Mittel und ihre Verwendung als Schädlingsbekämpfungsmittel.

(30) Priorität: **10.03.89 DE 3907784**

(43) Veröffentlichungstag der Anmeldung:
**12.09.90 Patentblatt 90/37**

(45) Bekanntmachung des Hinweises auf die Patenterteilung:
**15.02.95 Patentblatt 95/07**

(84) Benannte Vertragsstaaten:
**AT BE CH DE ES FR GB IT LI NL SE**

(56) Entgegenhaltungen:
**EP-A- 0 211 561**
**EP-A- 0 240 978**
**EP-A- 0 276 558**
**EP-A- 0 288 810**
**WO-A-88/08416**
**GB-A- 2 187 452**

(73) Patentinhaber: **HOECHST AKTIENGESELL-SCHAFT**

**D-65926 Frankfurt (DE)**

(72) Erfinder: **Schnatterer, Stefan, Dr.**
**Flurscheideweg 11**
**D-6230 Frankfurt am Main 80 (DE)**
Erfinder: **Schubert, Hans Herbert, Dr.**
**Geisenheimer Strasse 95**
**D-6000 Frankfurt am Main 71 (DE)**
Erfinder: **Salbeck, Gerhard, Dr.**
**Königsberger Strasse 52**
**D-6239 Kriftel/Taunus (DE)**
Erfinder: **Knauf, Werner, Dr.**
**Im Kirschgarten 24**
**D-6239 Eppstein/Taunus (DE)**
Erfinder: **Waltersdorfer, Anna, Dr.**
**Rauenthaler Weg 28**
**D-6000 Frankfurt am Main 71 (DE)**
Erfinder: **Kern, Manfred, Dr.**
**Im Traminer Weg 8**
**D-6501 Lörzweiler (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 386 715 B1

## Beschreibung

Es ist bekannt, daß bestimmte 1,4-Diarylbutan- und 1,4-Diarylbutenverbindungen insektizide oder akarizide Eigenschaften besitzen (GB 2,187,452 A oder Chem. Abstr. Vol. 109,73119z). Diese besitzen jedoch teilweise unzureichende Wirksamkeiten.

Aus WO-A-88 08416 sind 1,4-Diaryl-1-cyclopropyl-butan-Derivate sowie entsprechende Oxa- und Thia-Verbindungen und deren Verwendung als Insektizide und Akarizide bekannt.

In EP-A-288 810 werden 1,4-Diheteroaryl-butan-Derivate sowie entsprechende Oxa-Verbindungen und deren insektizide und akarizide Eigenschaften beschrieben.

Durch die Einführung von Heteroarylgruppen in die Arylbuten- und Arylbutanstruktur konnten neue Arylbuten- und Arylbutanderivate mit vorteilhaften schädlingsbekämpfenden, insbesondere insektiziden und/oder akariziden, Eigenschaften hergestellt werden.

Gegenstand der vorliegenden Erfindung sind daher die Verbindungen der Formeln I und II, deren Stereoisomere sowie die Gemische derselben

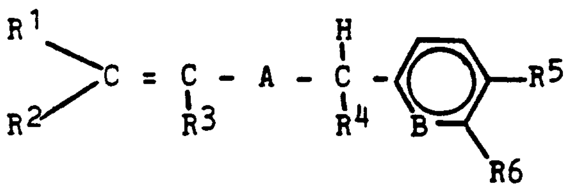

(I),

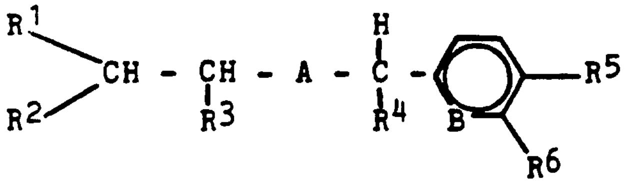

(II),

worin

$R^1$ = Pyridyl, Pyrimidyl, Thienyl, Thiazolyl, Furanyl, Pyrazolyl, 1,3-Oxazolyl oder Imidazolyl, die alle substituiert sein können,
$R^2$ = $(C_1\text{-}C_4)$Haloalkyl,
$R^3$ = H oder $(C_1\text{-}C_4)$Alkyl,
$R^4$ = H, $(C_1\text{-}C_4)$Alkyl, -CN oder -C≡CH,
A = $CH_2$, O oder S,
B = CH, N oder $C(CH_3)$,
$R^5$ = H oder Halogen und
$R^6$ = Phenoxy oder Benzyl, welche durch Halogen oder $(C_1\text{-}C_3)$Halogenalkyl ein- bis fünffach substituiert sein können, bedeuten.

Bevorzugte Verbindungen der Formeln I und II sind solche, bei denen

$R^1$ = einen Rest der Formeln A, B, C, D, E oder F

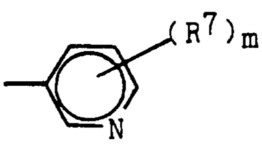 (A), 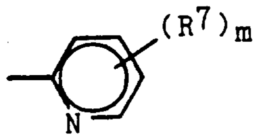 (B), 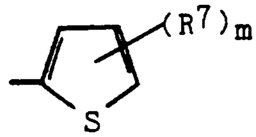 (C), 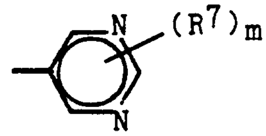 (D),

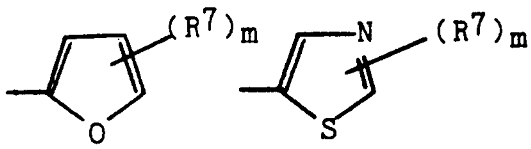

(E), (F)

mit $R^7$ = H, Halogen, $(C_1\text{-}C_4)$Alkyl, $(C_1\text{-}C_4)$Alkoxy, $(C_1\text{-}C_4)$Haloalkyl, $(C_1\text{-}C_4)$Haloalkoxy und m = 1 oder 2,

$R^2$ = $CF_3$,

$R^3$ = H oder Methyl,

B = CH,

$R^4$ = H, Methyl, -CN oder -C≡CH und

$R^6$ = Phenoxy, das in 4-Stellung durch Cl, Br oder $CF_3$ substituiert sein kann, bedeuten.

Insbesondere bevorzugt sind die Verbindungen der Formeln I und II, bei denen

$R^7$ = Cl, Br, $(C_1\text{-}C_3)$Alkoxy, $-OCF_3$, $-OCH_2CF_3$ oder $-OCF_2CF_2H$ bedeutet.

Für den Substituenten $R^1$ stehen bevorzugt die Reste der Formeln A, B oder C, insbesondere A.

Die Erfindung umfaßt alle Stereoisomeren, die bei den Formeln I und II auftreten können, insbesondere die Z und E-Diastereomeren der Olefinfunktion in Formel I sowie deren Gemische, ferner die einzelnen Enantiomeren sowie Enantiomerenpaare der Formeln I und II. Alkyl bedeutet sowohl geradkettiges als auch verzweigtes Alkyl.

Gegenstand der vorliegenden Erfindung ist ferner ein Verfahren zur Herstellung von Verbindungen der Formeln I und II, dadurch gekennzeichnet, daß man

a) für Verbindungen der Formel I eine Verbindung der Formel III

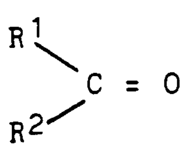 (III),

wobei $R^1$ und $R^2$ die Bedeutungen wie in Formel I besitzen, mit einer Verbindung der Formel IV

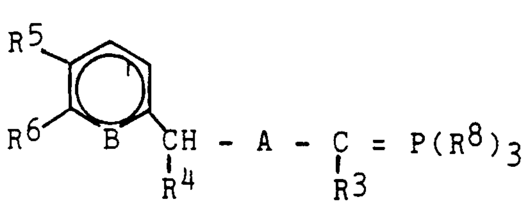 (IV),

wobei $R^3$, $R^4$, $R^5$, $R^6$, A und B die Bedeutungen wie in Formel I besitzen und $R^8$ = Phenyl, Cyclohexyl oder Butyl bedeutet, umsetzt und

b) für Verbindungen der Formel II die Olefinfunktion der entsprechenden Verbindung der Formel I mit Wasserstoff oder einer Wasserstoff abspaltenden Verbindung katalytisch hydriert.

Das unter a) beschriebene Verfahren ist als Wittig-Reaktion bekannt. Die Durchführung sowie die Reaktionsparameter sind in der Literatur beschrieben (H.J. Bestmann, Topics in Current Chemistry 109 (1983); Houben-Weyl, Methoden der organ. Chemie Band E1 und 5/1b). Die Carbonylfunktion der Verbindung III ist gegen den Rest $=P(R^8)_3$ der Verbindung IV und umgekehrt austauschbar.

Ferner läßt sich der Rest $R^1$ über eine Organometallverbindung in eine entsprechende Carbonylverbindung einführen. Aus dem gebildeten tertiären Alkohol entsteht dann durch Wasserabspaltung die Verbindung der Formel I (Houben-Weyl Band 6/1a/2).

Die für die Wittig-Reaktion a) als Ausgangsstoffe dienenden Ketone III werden durch Friedel-Crafts Acylierung von genügend elektronenreichen Aromaten erhalten (Houben-Weyl, Band 7/2a, 39, 83) oder aber durch Aufbau des Kohlenstoffgerüstes durch die Umsetzung von Arylmetallverbindungen mit geeigneten Carbonsäurederivaten wie Carbonsäurehalogeniden, Anhydriden, Estern oder Nitrilen (Houben-Weyl 7/2a, 548-621; X. Creary, J. Org. Chem. 52, 5026-30 (1987)).

Die Ylidverbindungen IV entstehen aus den entsprechenden Phosphoniumsalzen oder Phosphonestern durch Deprotonierung mit starken Basen wie Alkalihydriden, Organolithiumverbindungen, Alkaliamiden und Alkalialkoholaten in Ethern oder aromatischen Kohlenwasserstoffen als Lösungsmittel, entweder vor der Keton-Zugabe oder in Gegenwart des Ketons III bei Temperaturen von -20 °C bis +100 °C (Houben-Weyl, Band E1 und 5/1b).

Die Phosphoniumsalze V werden erhalten durch

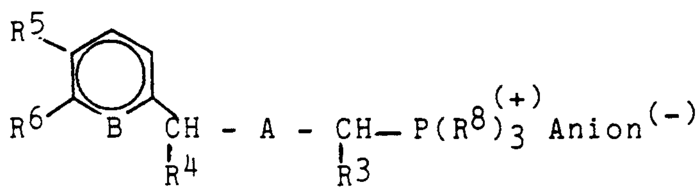

(V)

die Alkylierung von Triphenylphosphan oder Trialkylphosphan mit Aryl-alkylhalogeniden der Formel VI bei literaturbekannten Reaktionsbedingungen (Houben-Weyl Band E1, 495-515 und 12/1, 79-124).

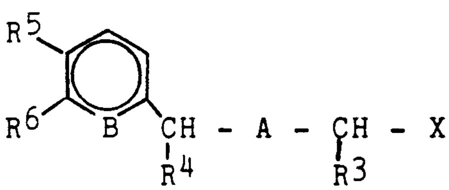

X = Cl, Br, J (VI)

Die Alkylierungsmittel VI entstehen einerseits, für $A = CH_2$, aus den entsprechenden 3-Aryl-propanolen VII und Halogenwasserstoff oder anorganischen Säurehalogeniden wie z.B. Thionylchlorid, andererseits werden sie, falls A = 0 oder S ist, aus dem entsprechenden Benzylalkohol und Formaldehyd und Halogenwasserstoff hergestellt (Houben-Weyl Band 5/3 und 5/4, DE-A-3125338). Die 3-Arylpropanole VII können durch Reduktion von Zimtsäuren und deren Estern VIII mit Aluminiumwasserstoffverbindungen wie z.B. Lithiumalanat dargestellt werden (Houben-Weyl, Band 4/1d, 190-216).

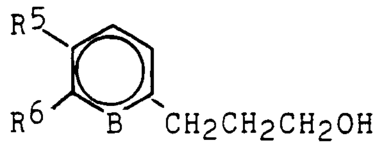

(VII)

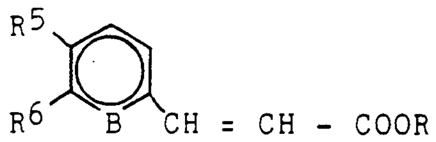

R' = H, Alkyl (VIII)

Die Synthese der Arylbutanverbindungen der Formel II erfolgt durch die Hydrierung der Olefinfunktion in einem Arylbuten der Formel I mit Wasserstoff oder einer Wasserstoff abspaltenden Verbindung wie z.B. Hydrazin, Cyclohexen oder Ameisensäurederivate unter dem katalytischen Einfluß von Palladium-, Platin-, Nickel- oder Rhodiumverbindungen bei literaturbekannten Reaktionsbedingungen (Houben-Weyl, Band 4/1c).

Ferner können die Verbindungen II durch Addition von Diboran, Boranaddukten oder alkylsubstituierten Boranen an die C,C-Doppelbindung von I und anschließender Hydrolyse der Bor-Kohlenstoffbindung mit Wasser oder Alkoholen hergestellt werden (Houben-Weyl, Band 4/1d, S. 49-59).

Die Wirkstoffe eignen sich bei guter Pflanzenverträglichkeit und günstiger Warmblütertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen. Sie sind gegen normal sensible und resistente Arten sowie alle oder einzelne Entwicklungsstadien wirksam Zu den oben erwähnten Schädlingen gehören:

Aus der Ordnung der Acarina z.B. Acarus siro, Agras spp., Ornithrodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp.

Aus der Ordnung der Isopoda, z.B. Oniscus asellus, Armadium vulgare, Porcellio scaber.

Aus der Ordnung der Diplopoda z.B. Blaniulus guttulatus.

Aus der Ordnung der Chilopoda z.B. Geophilus carpophagus, Scutigera spp.

Aus der Ordnung der Symphyla z.B. Scutigerella immaculata.

Aus der Ordung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregarai.

Aus der Ordnung der Dermaptera z.B. Forficula auricularia.

Aus der Ordnung des Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporarium, Aphis gossypii, Brevicornyne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelus bilobatus, Naphotettix cincticeps, Lecanium corni, Saissetia oleae, Laodelphax striatellus, Nilaparvata lugens, Aonidiella auroantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia

spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cocoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliophora erythrocephala, Lucilia spp., Chrysomyia spp., Cuterebra spp., Gastrophilus spp., Hypobosca spp., Stomoxys spp., Oestrus spp. Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopsis, Ceratophyllus spp.

Aus der Ordnung der Arachnida z.B. Scorpio maurus, Latrodectus mactans.

Aus der Klasse der Helminthen z.B. Haemonchus, Trichostrongulus, Ostertagia, Cooperia, Chabertia, Strongyloides, Oesophagostomum, Hyostrongulus, Ancylostoma, Ascaris und Heterakis sowie Fasciola und pflanzenschädigende Nematoden z.B. solche der Gattungen Meloidogyne, Heterodera, Ditylenchus, Aphelenchoides, Radopholus, Globodera, Pratylenchus, Longidorus und Xiphinema.

Aus der Klasse der Gastropoda z.B. Deroceras spp., Arion spp., Lymnaea spp., Galba spp., Succinea spp., Biophalaria spp., Bulinus spp., Oncomelania spp.

Aus der Klasse der Bivalva z.B. Dreissena spp.

Gegenstand der Erfindung sind auch Mittel, die die Verbindungen der Formeln I und II neben geeigneten Formulierungshilfsmitteln enthalten.

Die erfindungsgemäßen Mittel enthalten die Wirkstoffe der Formeln I und II im allgemeinen zu 1 bis 95 Gew.-%.

Sie können auf verschiedene Art formuliert werden, je nachdem wie es durch die biologischen und/oder chemisch-physikalischen Parameter vorgegeben ist. Als Formulierungsmöglichkeiten kommen daher infrage: Spritzpulver (WP), emulgierbare Konzentrate (EC), wäßrige Lösungen (SC), Emulsionen, versprühbare Lösungen, Dispersionen auf Öl- oder Wasserbasis (SC), Suspoemulsionen (SC), Stäubemittel (DP), Beizmittel, Granulate in Form von Mikro-, Sprüh-, Aufzugs- und Adsorptionsgranulaten, wasserdispergierbare Granulate (WC), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten. Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzol-sulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen, durch Mikroorganismen hergestellte Stoffe u.a.
Bevorzugte Mischungspartner sind

1. aus der Gruppe der Phosphorverbindungen
Acephate, Azamethiphos, Azinphos-ethyl, Azinphos-methyl, Bromophos, Bromophos-ethyl, Chlorfenvinphos, Chlormephos, Chlorpyrifos, Chlorpyrifos-methyl, Demeton, Demeton-S-methyl, Demeton-S-methyl sulfphone, Dialifos, Diazinon, Dichlorvos, Dicrotophos, O,O-1,2,2,2-tetrachlorethylphosphorthioate (SD 208 304), Dimethoate, Disulfoton, EPN, Ethion, Ethoprophos, Etrimfos, Famphur, Fenamiphos, Fenitriothion, Fensulfothion, Fenthion, Fonofos, Formothion, Heptenophos, Isozophos, Isothioate, Isoxathion, Malathion, Methacrifos, Methamidophos, Methidathion Salithion, Mevinphos, Monocrotophos, Naled, Omethoate, Oxydemeton-methyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tetrachlorvinphos, Thiometon, Triazophos, Trichlorphon, Vamidothion;

2. aus der Gruppe der Carbamate
Aldicarb, 2-sec.-Butylphenylmethylcarbamate (BPMC), Carbaryl, Carbofuran, Carbosulfan, Cloethocarb, Benfuracarb, Ethiofencarb, Furathiocarb, Isoprocarb, Methomyl, 5-Methyl-m-cu-menylbutyryl(methyl)-carbamate, Oxamyl, Pirimicarb, Propoxur, Thiodicarb, Thiofanox, Ethyl 4,6,9-triaza-4-benzyl-6,10-dimethyl-8-oxa-7-oxo-5,11-dithia-9-dodecenoate (OK 135), 1-methylthio(ethylideneamino)-N-methyl-N-(morpholinothio)carbamate (UC 51717);

3. aus der Gruppe der Carbonsäureester
Allethrin, Alphametrin, 5-Benzyl-3-furylmethyl-(E)-(1R)-cis-2,2-di-methyl-3-(2-oxothiolan-3-ylidenemethyl)-cyclopropanecarboxylate, Bioallethrin, Bioallethrin((S)-cyclopentylisomer), Bioresmethrin, Biphenate, (RS)-1-Cyano-1-(6-phenoxy-2-pyridyl)methyl-(1RS)-trans-3-(4-tert.butylphenyl)-2,2-dimethylcyclopropanecarboxylate (NCI 85193), Cycloprothrin, Cyfluthrin, Cyhalothrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-isomer), Permethrin, Pheothrin ((R)-Isomer), d-Pralethrin, Pyrethrine (natürliche Produkte), Resmethrin, Tefluthrin, Tetramethrin, Tralomethrin;

4. aus der Gruppe der Amidine
Amitraz, Chlordimeform;
5. aus der Gruppe der Zinnverbindungen
Cyhexatin, Fenbutatinoxide;
6. Sonstige
Abamectin, Bacillus thuringiensis, Bensultap, Binapacryl, Bromopropylate, Buprofezin, Camphechlor, Cartap, Chlorobenzilate, Chlorfluazuron, 2-(4-(Chlorphenyl)-4,5-di-phenylthiophen (UBI-T 930), Chlorfentezine, Cyclopropancarbonsäure-(2-naphthylmethyl)ester (Ro 12-0470), Cyromazin, N-(3,5-Dichlor-4-(1,1,2,3,3,3-hexafluor-1-propyloxy)phenyl)carbamoyl)-2-chlorbenzcarboximidsäureethylester, DDT, Dicofol, N-(N-(3,5-Di-chlor-4-(1,1,2,2-tetrafluorethoxy)phenylamino)carbonyl)-2,6-difluorbenzamid (XRD 473), Diflubenzuron, N-(2,3-Dihydro-3-methyl-1,3-thiazol-2-ylidene)-2,4-xylidine, Dinobuton, Dinocap, Endosulfan, Ethofenprox, (4-Ethoxyphenyl) (dimethyl)(3-(3-phenoxyphenyl)propyl)silan, (4-Ethoxyphenyl) (3-(4-fluouo-3-phenoxyphenyl)propyl)dimethylsilan, Fenoxycarb, 2-Fluoro-5-(4-(4-ethoxyphenyl)-4-methyl-1-pentyl)diphenylether (MTI 800), Granulose- und Kernpolyederviren, Fenthiocarb, Flubenzimine, Flucycloxuron, Flufenoxuron, Gamma-HCH, Hexythiazox, Hydramethylnon (AC 217300), Ivermectin, 2-Nitromethyl-4,5-dihydro-6H-thiazin (SD 52618), 2-Nitromethyl-3,4-dihydrothiazol (SD 35651), 2-Nitromethylene-1,2-thiazinan-3-ylcarbamaldehyde (WL 108477), Propargite, Teflubenzuron, Tetradifon, Tetrasul, Thiocyclam, Triflumuron.

Der Wirkstoffgehalt der aus den handelsüblichen Formulierungen bereiteten Anwendungsformen kann von 0,00000001 bis zu 100 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Endo- und Ektoparasiten auf dem veterinärmedizinischen Gebiet bzw. auf dem Gebiet der Tierhaltung.

Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die neuen Verbindungen können bei Rindern z.B. in Dosierungen von 0,01 bis 1 mg/kg Körpergewicht eingesetzt werden.

Nachfolgende Beispiele dienen zur Erläuterung der Erfindung.

A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

B. Chemische Beispiele

1,1,1-Trifluor-2-(2-ethoxy-5-pyridyl)-5-(3-phenoxyphenyl)-2-penten (Nr. 1, Beispiel für Formel I)

In 50 ml Toluol unter Schutzgas werden 2,5 g (11,4 mmol) 2-Ethoxy-5-trifluoracetylpyridin, 6,0 g (10,8 mmol) 3-(3-Phenoxyphenyl)propyl-triphenylposphoniumbromid und 1,3 g (12,0 mmol) Kalium-tert-butylat vermischt und 6 h lang bei 20 °C gerührt. Danach wird mit Wasser das Kaliumbromid ausgewaschen, die Toluol-Phase eingeengt und der Toluol-Extrakt durch Säulenchromatographie (Laufmittel: Essigester-Heptan 2:8; stationäre Phase Kieselgel) aufgetrennt. Man erhält 3,50 g (75 %) des Olefin-Produktes (1) als farblose Flüssigkeit. Löslichkeit: 11. Heptan, Toluol; ul. Wasser. Die $^{1}$H-NMR- und GC-Messungen bestätigen das Vorliegen eines Diastereomerengemisches (Z und E Isomeres) im Verhältnis 2,3:1, Kp.1013 386 und 395 °C (GC-Messung).

1,1,1-Trifuor-2-(2-ethoxy-5-pyridyl)-5-(3-phenoxyphenyl)-pentan (Nr. 61, Beispiel für Formel II)

In 100 ml Ethanol wird 1,0 g Platin auf Aktivkohle (10 %ig) unter einer Wasserstoffatmosphäre vorgelegt und 5,0 g (12,1 mmol) Olefin (1) zugegeben. Nach 4 h bei 1013 mbar wird die Reaktionsmischung durch Kieselgel filtriert und eingeengt. Man erhält die Verbindung Nr. 61 als farblose Flüssigkeit, die Ausbeute ist 4,9 g (97 %).

Gemäß diesen Vorschriften lassen sich die nachfolgend genannten Verbindungen der Formeln I und II herstellen.

Abkürzungen: Me = $CH_3$, Et = $C_2H_5$, Ph = $C_6H_5$, iPr = $CH(CH_3)_2$

Tabelle 1

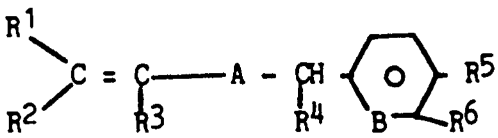

(I)

| Bsp. Nr. | $R^1$ | $R^2$ | $R^3$ | A | $R^4$ | B | $R^5$ | $R^6$ | physikal. Daten (Kp. °C, GC/ 1013 mbar) |
|---|---|---|---|---|---|---|---|---|---|
| 1 | 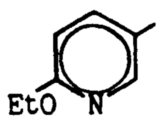 | $CF_3$ | H | $CH_2$ | H | CH | H | OPh | 386, 395 |
| 2 | " | $CF_3$ | H | O | H | CH | H | OPh | 393, 401 |
| 3 | " | $CF_3$ | H | S | H | CH | H | OPh | |
| 4 | " | $CF_3$ | H | $CH_2$ | H | CH | F | OPh | |
| 5 | " | $CF_3$ | H | O | H | CH | F | OPh | 389, 397 |
| 6 | 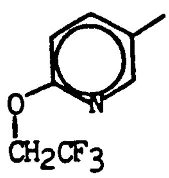 | $CF_3$ | H | $CH_2$ | H | CH | H | OPh | 372, 382 |
| 7 | " | $CF_3$ | H | O | H | CH | H | OPh | 377, 387 |
| 8 | " | $CF_3$ | H | $CH_2$ | H | CH | F | OPh | 374, 385 |
| 9 | " | $CF_3$ | H | O | H | CH | F | OPh | 381, 390 |

Fortsetzung Tabelle 1

| Bsp. Nr. | $R^1$ | $R^2$ | $R^3$ | A | $R^4$ | B | $R^5$ | $R^6$ | physikal. Daten (Kp. °C, GC/ 1013 mbar) |
|---|---|---|---|---|---|---|---|---|---|
| 10 | 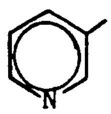 | $CF_3$ | H | $CH_2$ | H | CH | H | OPh | |
| 11 | " | $CF_3$ | H | O | H | CH | H | OPh | |
| 12 | 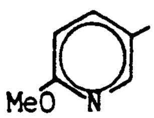 | $CF_3$ | H | $CH_2$ | H | CH | H | OPh | |
| 13 | " | $CF_3$ | H | O | H | CH | H | OPh | |
| 14 | 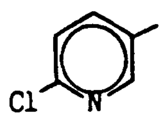 | $CF_3$ | H | $CH_2$ | H | CH | H | OPh | |
| 15 | " | $CF_3$ | H | O | H | CH | H | OPh | |
| 16 | 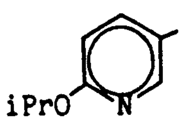 | $CF_3$ | H | $CH_2$ | H | CH | H | OPh | |
| 17 | " | $CF_3$ | H | O | H | CH | H | OPh | |
| 18 | " | $CF_3$ | H | $CH_2$ | H | CH | F | OPh | |
| 19 | " | $CF_3$ | H | O | H | CH | F | OPh | |

Fortsetzung Tabelle 1

| Bsp. Nr. | $R^1$ | $R^2$ | $R^3$ | A | $R^4$ | B | $R^5$ | $R^6$ | physikal. Daten (Kp. °C, GC/ 1013 mbar) |
|---|---|---|---|---|---|---|---|---|---|
| 20 | 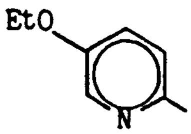 | $CF_3$ | H | $CH_2$ | H | CH | H | OPh | |
| 21 | " | $CF_3$ | H | O | H | CH | H | OPh | |
| 22 | 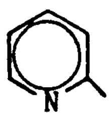 | $CF_3$ | H | $CH_2$ | H | CH | H | OPh | |
| 23 | " | $CF_3$ | H | O | H | CH | H | OPh | |
| 24 | 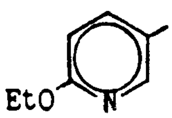 | $CF_3$ | $CH_3$ | $CH_2$ | H | CH | H | OPh | |
| 25 | " | $CF_3$ | $CH_3$ | $CH_2$ | H | CH | F | OPh | |
| 26 | " | $CF_3$ | H | O | CN | CH | H | OPh | |
| 27 | 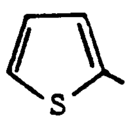 | $CF_3$ | H | $CH_2$ | H | CH | H | OPh | |
| 28 | " | $CF_3$ | H | O | H | CH | H | OPh | |
| 29 | 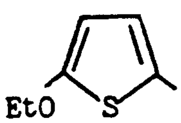 | $CF_3$ | H | $CH_2$ | H | CH | H | OPh | |
| 30 | " | $CF_3$ | H | O | H | CH | H | OPh | |

Fortsetzung Tabelle 1

| Bsp. Nr. | $R^1$ | $R^2$ | $R^3$ | A | $R^4$ | B | $R^5$ | $R^6$ | physikal. Daten (Kp. °C, GC/ 1013 mbar) |
|---|---|---|---|---|---|---|---|---|---|
| 31 | 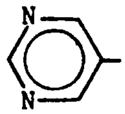 | $CF_3$ | H | $CH_2$ | H | CH | H | OPh | |
| 32 | " | $CF_3$ | H | O | H | CH | H | OPh | |
| 33 | 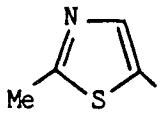 | $CF_3$ | H | $CH_2$ | H | CH | H | OPh | |
| 34 | " | $CF_3$ | H | O | H | CH | H | OPh | |
| 35 | 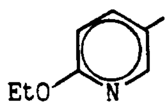 | $CF_3$ | H | $CH_2$ | H | N | H | OPh | |
| 36 | " | $CF_3$ | H | O | H | N | H | OPh | |
| 37 | 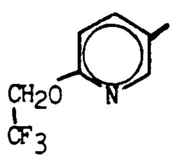 | $CF_3$ | H | $CH_2$ | H | N | H | OPh | |
| 38 | " | $CF_3$ | H | O | H | N | H | OPh | |

$^1$H-NMR-Daten (LM: $CDCl_3$, Standard : TMS, $\delta$(H) [ppm])

4

E-Olefin: 1,39, t (3H, $OCH_2CH_3$, 7.3 Hz); 2,30, m (2H, $CH_2CH_2CH$); 2,61, t (2H, $ArCH_2CH_2$, 7.0 Hz); 4,36, q, (2H, $OCH_2CH_3$, 7.3 Hz); 6,42 tq (1H, $C=CHCH_2$, 1.7 Hz, 7.5 Hz); 6,68-7,35, m (10H, Aryl-H); 7,86, d (1H, 6-Pyridyl-H, 2.5 Hz).

Z-Olefin: 1,38, t (3H, $OCH_2CH_3$, 7.3 Hz); 2,73, m (4H, $ArCH_2CH_2CH$); 4,36, q (2H, $OCH_2CH_3$, 7.3 Hz); 5,95, tq (1H, $C=CHCH_2$, 6,9 Hz); 6,65, d (1H, 3-Pyridyl-H, 8.8 Hz); 6,85-7,48, m (10H, Aryl-H); 7,99, d (1H, 6-Pyridyl-H, 2.5 Hz).

Tabelle 2

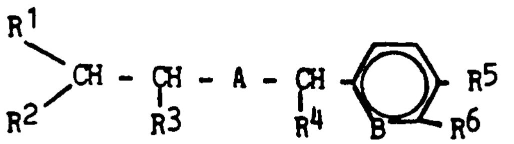

(II)

| Bsp. Nr. | $R^1$ | $R^2$ | $R^3$ | A | $R^4$ | B | $R^5$ | $R^6$ | physikal. Daten (Kp. °C, GC/ 1013 mbar) |
|---|---|---|---|---|---|---|---|---|---|
| 39 | 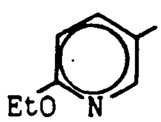 | $CF_3$ | H | $CH_2$ | H | CH | H | OPh | 392 |
| 40 | " | $CF_3$ | H | O | H | CH | H | OPh | 390 |
| 41 | " | $CF_3$ | H | O | H | CH | F | OPh | 385 |
| 42 | " | $CF_3$ | H | $CH_2$ | H | CH | F | OPh | 394 |
| 43 | " | $CF_3$ | $CH_3$ | $CH_2$ | H | CH | H | OPh | |
| 44 | " | $CF_3$ | $CH_3$ | $CH_2$ | H | CH | F | OPh | |
| 45 | " | $CF_3$ | H | O | CN | CH | H | OPh | |
| 46 | 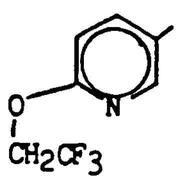 | $CF_3$ | H | $CH_2$ | H | CH | H | OPh | 380 |
| 47 | " | $CF_3$ | H | O | H | CH | H | OPh | 382 |
| 48 | " | $CF_3$ | H | $CH_2$ | H | CH | F | OPh | 384 |
| 49 | " | $CF_3$ | H | O | H | CH | F | OPh | 378 |

Fortsetzung Tabelle 2

| Bsp. Nr. | $R^1$ | $R^2$ | $R^3$ | A | $R^4$ | B | $R^5$ | $R^6$ | physikal. Daten (Kp. °C , GC/ 1013 mbar) |
|---|---|---|---|---|---|---|---|---|---|
| 50 | 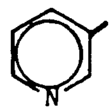 | $CF_3$ | H | $CH_2$ | H | CH | H | OPh | |
| 51 | " | $CF_3$ | H | O | H | CH | H | OPh | |
| 52 | 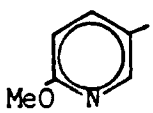 | $CF_3$ | H | $CH_2$ | H | CH | H | OPh | |
| 53 | " | $CF_3$ | H | O | H | CH | H | OPh | |
| 54 | 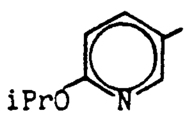 | $CF_3$ | H | $CH_2$ | H | CH | H | OPh | |
| 55 | " | $CF_3$ | H | O | H | CH | H | OPh | |
| 56 | " | $CF_3$ | H | $CH_2$ | H | CH | F | OPh | |
| 57 | " | $CF_3$ | H | O | H | CH | F | OPh | |
| 58 | 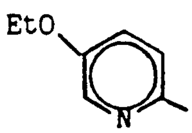 | $CF_3$ | H | $CH_2$ | H | CH | H | OPh | |
| 59 | " | $CF_3$ | H | O | H | CH | H | OPh | |
| 60 | 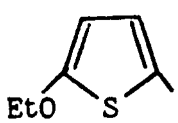 | $CF_3$ | H | $CH_2$ | H | CH | H | OPh | |
| 61 | " | $CF_3$ | H | O | H | CH | H | OPh | |
| 62 | 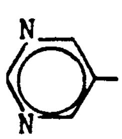 | $CF_3$ | H | $CH_2$ | H | CH | H | OPh | |
| 63 | " | $CF_3$ | H | O | H | CH | H | OPh | |

Fortsetzung Tabelle 2

| Bsp. Nr. | $R^1$ | $R^2$ | $R^3$ | A | $R^4$ | B | $R^5$ | $R^6$ | physikal. Daten (Kp. °C , GC/ 1013 mbar) |
|---|---|---|---|---|---|---|---|---|---|
| 64 | 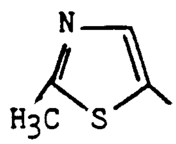 | $CF_3$ | H | $CH_2$ | H | CH | H | OPh | |
| 65 | " | $CF_3$ | H | O | H | CH | H | OPh | |
| 66 | 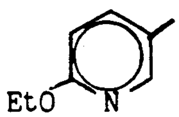 | $CF_3$ | H | $CH_2$ | H | N | H | OPh | |
| 67 | " | $CF_3$ | H | O | H | N | H | OPh | |
| 68 | 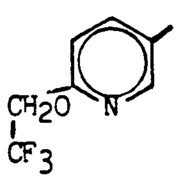 | $CF_3$ | H | $CH_2$ | H | N | H | OPh | |
| 69 | " | $CF_3$ | H | O | H | N | H | OPh | |
| 70 | 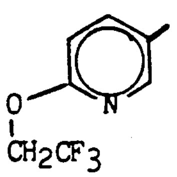 | $CF_3$ | H | O | H | CH | H | OPh | 390 |
| 71 | 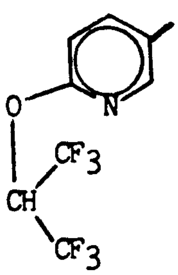 | $CF_3$ | H | $CH_2$ | H | CH | F | OPh | |
| 72 | " | $CF_3$ | H | O | H | CH | F | OPh | |

$^1$H-NMR-Daten (LM: $CDCl_3$, Standard: TMS, $\delta$(H) [ppm])

46

1,48, m (2H, $CH_2$); 1,90, m (2H, $CH_2$); 2,56, t ($ArCH_2$, 2H, 7.3 Hz); 3,19, m (1H, $F_3CCH$); 4,73, q (2H, $OCH_2CF_3$, 8,6 Hz); 6,75-7,36, m (10H, Aryl-H); 7,51, dd (1H, 4-Pyridyl-H, 8.8 Hz, 2.5 Hz); 7,98, d (1H, 6-Pyridyl-H, 2.5 Hz).

C. Biologische Beispiele

Beispiel 1

Auf die Innenseite des Deckels und des Bodens einer Petrischale werden jeweils 1 ml der zu testenden Formulierung emulgiert in Wasser gleichmäßig aufgetragen und nach dem Antrocknen des Belages jeweils 10 Imagines der Hausfliege (Musca domestica) eingegeben. Nach dem Verschließen der Schale wird diese bei Raumtemperatur aufbewahrt und nach 3 Stunden die Mortalität der Versuchstiere bestimmt. Bei 250 ppm (bezogen auf den Gehalt an Wirkstoff) zeigen die Präparate 1, 2, 6, 7, 39, 42 und 46 eine gute Wirkung (100 % Mortalität) gegenüber der Stubenfliege.

Beispiel 2

In die wie in Beispiel 1 behandelte Petrischale werden je 10 Larven (L4) der Deutschen Schabe (Blattella germanica) gesetzt, die Schalen verschlossen und nach 5 Tagen die Mortalität der Versuchstiere bestimmt.

Bei 100 ppm (bezogen auf den Gehalt an Wirkstoff) zeigten die Präparate 1, 2, 6, 39 und 46 eine gute Wirkung (100 % Mortalität) gegenüber der Deutschen Schabe.

Beispiel 3

Auf der Innenseite des Bodens einer Petrischale wird 1 Lage Filterpapier und darauf eine 3 - 5 ml große Menge halbsynthetische Futterdiät aufgebracht. Nach dem Erkalten wird die zu testende Formulierung mit Wasser auf die Oberfläche des Futters und des Filterpapiers in fallenden Konzentrationen gesprüht und nach dem Antrocknen des Spritzbelages je 10 Larven (L3 - L4) des gemeinen Baumwollwurms (Prodenia litura) eingesetzt.

Die mit Deckel verschlossenen Petrischalen werden 7 Tage bei Raumtemperatur aufbewahrt und danach die Mortalität der Versuchstiere ermittelt.

Bei 1000 ppm (bezogen auf den Gehalt an Wirkstoff) zeigen die Präparate 1, 2, 6, 7, 39, 42 und 46 eine gute Wirkung gegen Larven des gemeinen Baumwollwurms.

Beispiel 4

Larven des mexikanischen Bohnenkäfers (Epilachna varivestis, L III) wurden in einer Spritzapparatur mit einer Wirkstoffzubereitung der gewünschten Konzentration behandelt. Gleichzeitig wurden Buschbohnenblätter (Phaseolus vulgaris) in die entsprechende Wirkstofflösung getaucht. Nach dem Antrocknen des Spritzbelages wurden die Larven des Käfers auf die Bohnenblätter gesetzt. Nach 3 Tagen wurde die Abtötung der Larven in Prozent bestimmt. Bei 1000 ppm (bezogen auf Wirkstoff) zeigten die Präparate 1, 2, 6, 7, 39, 42 und 46 eine gute Wirkung gegenüber mexikanischem Bohnenkäfer.

Beispiel 5

Larven des letzteren Larvenstadiums der Reiszikade (Nilaparvata lugens) werden in Petrischalen gesetzt, deren Boden mit einem behandelten saugfähigen Papier ausgelegt sind. Vor dem Einsetzen der Tiere wird jedes Filterpapier mit 2 ml dest. Wasser angefeuchtet und dieses anschließend mit fallenden Dosierungen des Versuchspräparates emulgiert in Wasser entsprechend 600 l Wasser/ha besprüht. Nach dem Besetzen der Schalen werden diese sofort verschlossen und bis zur Auswertung 24 h unter Laborbedingungen aufbewahrt. Danach wird die Wirkung (% Mortalität) ausgewertet. Die Präparate 1 und 42 zeigen bei einer Wirkungskonzentration in der Spritzbrühe von 125 ppm eine 100 %ige Wirkung gegenüber Reiszikaden.

Beispiel 6

Mit Kundebohnenblattlaus (Aphis craccivora) stark besetzte Ackerbohnen (Vicia faba) werden mit wäßrigen Verdünnungen von Spritzpulverkonzentraten mit 100 ppm Wirkstoffgehalt bis zum Stadium des beginnenden Abtropfens besprüht. Die Mortalität der Blattläuse wird nach 3 Tagen bestimmt. Eine 100 %ige Abtötung kann mit den Verbindungen gemäß Beispielen 1, 6, 7, 39, 42 und 46 erzielt werden.

**Patentansprüche**
**Patentansprüche für folgende Vertragsstaaten : AT, BE, CH, DE, FR, GB, IT, LI, NL, SE**

1. Verbindungen der Formeln I und II, deren Stereoisomere sowie die Gemische derselben

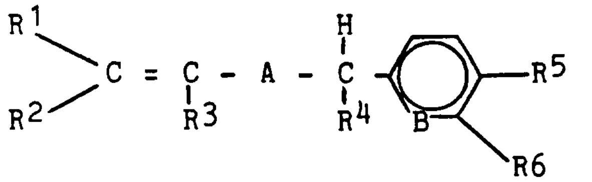 (I),

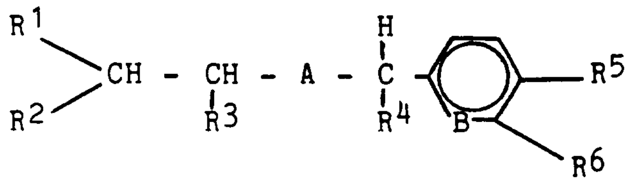 (II),

worin
- $R^1$ = Pyridyl, Pyrimidyl, Thienyl, Thiazolyl, Furanyl, Pyrazolyl, 1,3-Oxazolyl oder Imidazolyl, die alle substituiert sein können,
- $R^2$ = $(C_1\text{-}C_4)$Haloalkyl,
- $R^3$ = H oder $(C_1\text{-}C_4)$Alkyl,
- $R^4$ = H, $(C_1\text{-}C_4)$Alkyl, -CN oder -C≡CH,
- A = $CH_2$, O oder S,
- B = CH, N oder $C(CH_3)$,
- $R^5$ = H oder Halogen und
- $R^6$ = Phenoxy oder Benzyl, welche durch Halogen oder $(C_1\text{-}C_3)$Halogenalkyl ein- bis fünffach substituiert sein können, bedeuten.

2. Verbindungen der Formeln I und II von Anspruch 1, worin
   $R^1$ = einen Rest der Formeln A, B, C, D, E oder F

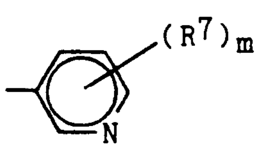 (A), 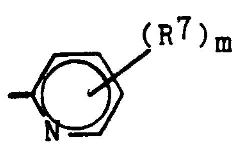 (B), 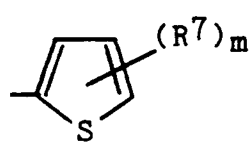 (C), 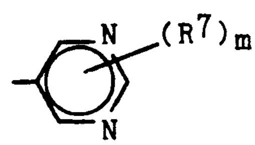 (D),

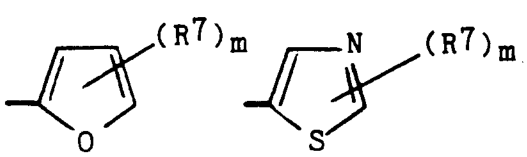 (E), (F)

mit $R^7$ = H, Halogen, $(C_1\text{-}C_4)$Alkyl, $(C_1\text{-}C_4)$Alkoxy, $(C_1\text{-}C_4)$Haloalkyl, $(C_1\text{-}C_4)$Haloalkoxy und m = 1 oder 2,

$R^2$ = $CF_3$,
$R^3$ = H oder Methyl,
B = CH,
$R^4$ = H, Methyl, -CN oder -C≡CH und
$R^6$ = Phenoxy, das in 4-Stellung durch Cl, Br oder $CF_3$ substituiert sein kann, bedeuten.

**3.** Verbindungen der Formeln I und II von Anspruch 2, worin
$R^7$ = Cl, Br, ($C_1$-$C_3$)Alkoxy, -$OCF_3$, -$OCH_2CF_3$ oder -$OCF_2CF_2H$ bedeutet.

**4.** Verbindungen gemäß einem oder mehreren der Ansprüche 1 bis 3, dadurch gekennzeichnet, daß $R^1$ einen Rest der Formeln A, B oder C bedeutet.

**5.** Verbindungen gemäß einem oder mehreren der Ansprüche 1 bis 4, dadurch gekennzeichnet, daß $R^1$ einen Rest der Formel A bedeutet.

**6.** Verfahren zur Herstellung von Verbindungen der Formeln I und II gemäß einem oder mehreren der Ansprüche 1 bis 5, dadurch gekennzeichnet, daß man
a) für Verbindungen der Formel I eine Verbindung der Formel III

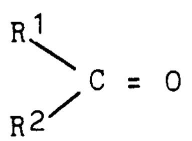

(III),

wobei $R^1$ und $R^2$ die Bedeutungen wie in Formel I besitzen, mit einer Verbindung der Formel IV

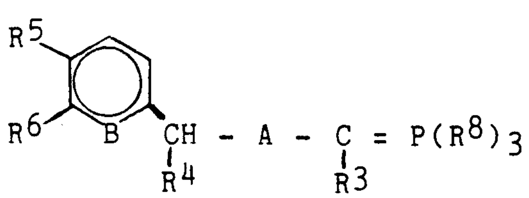

(IV),

wobei $R^3$, $R^4$, $R^5$, $R^6$, A und B die Bedeutungen wie in Formel I besitzen und $R^8$ = Phenyl, Cyclohexyl oder Butyl bedeutet, umsetzt und
b) für Verbindungen der Formel II die Olefinfunktion der entsprechenden Verbindung der Formel I mit Wasserstoff oder einer Wasserstoff abspaltenden Verbindung katalytisch hydriert.

**7.** Insektizide oder akarizide Mittel, dadurch gekennzeichnet, daß sie eine wirksame Menge einer Verbindung der Formeln I und/oder II enthalten.

**8.** Verwendung der Verbindungen der Formeln I und/oder II zur Bekämpfung von Schadinsekten oder Akariden.

**9.** Verfahren zur Bekämpfung von Schadinsekten oder Akariden, dadurch gekennzeichnet, daß man auf diese oder die von ihnen befallenen Pflanzen, Flächen oder Substrate eine wirksame Menge einer Verbindung der Formeln I und/oder II appliziert.

**Patentansprüche für folgenden Vertragsstaat : ES**

**1.** Verfahren zur Herstellung von Verbindungen der Formeln I und II, deren Stereoisomere sowie die Gemische derselben

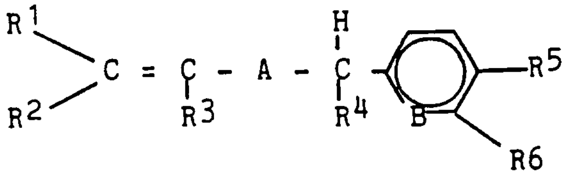

(I),

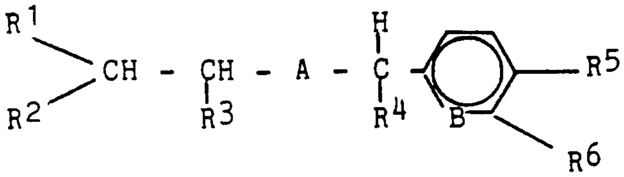

(II'),

worin

$R^1$ = Pyridyl, Pyrimidyl, Thienyl, Thiazolyl, Furanyl, Pyrazolyl, 1,3-Oxazolyl oder Imidazolyl, die alle substituiert sein können,
$R^2$ = $(C_1\text{-}C_4)$Haloalkyl,
$R^3$ = H oder $(C_1\text{-}C_4)$Alkyl,
$R^4$ = H, $(C_1\text{-}C_4)$Alkyl, -CN oder -C≡CH,
A = $CH_2$, O oder S,
B = CH, N oder $C(CH_3)$,
$R^5$ = H oder Halogen und
$R^6$ = Phenoxy oder Benzyl, welche durch Halogen oder $(C_1\text{-}C_3)$Halogenalkyl ein- bis fünffach substituiert sein können, bedeuten, dadurch gekennzeichnet, daß man

a) für Verbindungen der Formel I eine Verbindung der Formel III

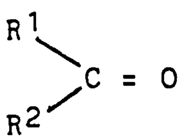

(III),

wobei $R^1$ und $R^2$ die Bedeutungen wie in Formel I besitzen, mit einer Verbindung der Formel IV

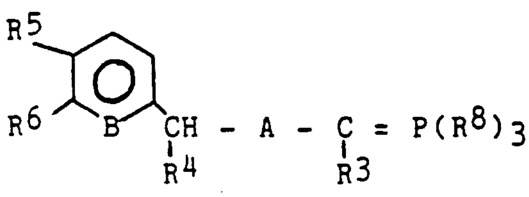

(IV),

wobei $R^3$, $R^4$, $R^5$, $R^6$, A und B die Bedeutungen wie in Formel I besitzen und $R^8$ = Phenyl, Cyclohexyl oder Butyl bedeutet, umsetzt und

b) für Verbindungen der Formel II die Olefinfunktion der entsprechenden Verbindung der Formel I mit Wasserstoff oder einer Wasserstoff abspaltenden Verbindung katalytisch hydriert.

**2.** Verfahren gemäß Anspruch 1, dadurch gekennzeichnet, daß in Formeln I und II

$R^1$ = einen Rest der Formeln A, B, C, D, E oder F

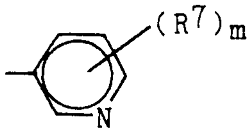
(A),

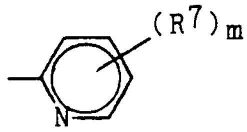
(B),

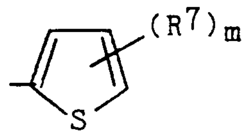
(C),

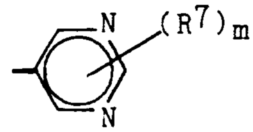
(D),

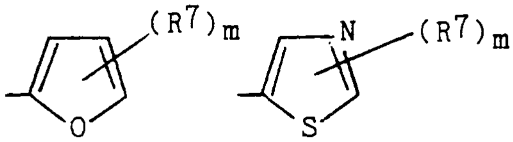
(E), (F)

mit $R^7$ = H, Halogen $(C_1\text{-}C_4)$Alkyl, $(C_1\text{-}C_4)$Alkoxy, $(C_1\text{-}C_4)$Haloalkyl, $(C_1\text{-}C_4)$Haloalkoxy und
m = 1 oder 2,
$R^2$ = $CF_3$,
$R^3$ = H oder Methyl,
B = CH,
$R^4$ = H, Methyl, -CN oder -C≡CH und
$R^6$ = Phenoxy, das in 4-Stellung durch Cl, Br oder $CF_3$ substituiert sein kann, bedeuten.

**3.** Verfahren gemäß Anspruch 1 oder 2, dadurch gekennzeichnet, daß in Formeln I und II
$R^7$ = Cl, Br, $(C_1\text{-}C_3)$Alkoxy, $-OCF_3$, $-OCH_2CF_3$ oder $-OCF_2CF_2H$ bedeutet.

**4.** Verfahren gemäß Ansprüchen 1 bis 3, dadurch gekennzeichnet, daß in Formeln I und II $R^1$ einen Rest der Formeln A, B oder C bedeutet.

**5.** Verfahren gemäß Anspruch 1 bis 4, dadurch gekennzeichnet, daß in Formeln I und II $R^1$ einen Rest der Formel A bedeutet.

**6.** Insektizide oder akarizide Mittel, dadurch gekennzeichnet, daß sie eine wirksame Menge einer Verbindung der Formeln I und/oder II gemäß Ansprüchen 1 bis 5 enthalten.

**7.** Verwendung der Verbindungen der Formeln I und/oder II gemäß Ansprüchen 1 bis 5 zur Bekämpfung von Schadinsekten oder Akariden.

**8.** Verfahren zur Bekämpfung von Schadinsekten oder Akariden, dadurch gekennzeichnet, daß man auf diese oder die von ihnen befallenen Pflanzen, Flächen oder Substrate eine wirksame Menge einer Verbindung der Formeln I und/oder II gemäß Ansprüchen 1 bis 5 appliziert.

**Claims**

**Claims for the following Contracting States : AT, BE, CH, DE, FR, GB, IT, LI, NL, SE**

**1.** A compound of the formulae I and II, the stereoisomers thereof, and the mixtures of the latter

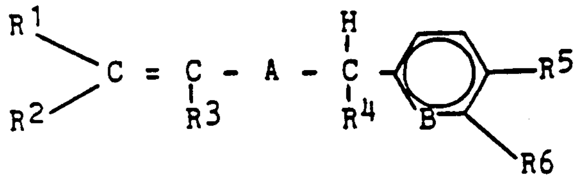

(I)

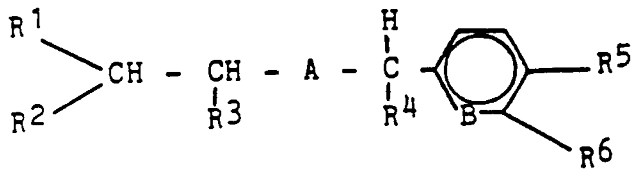

(II)

where

- $R^1$ is pyridyl, pyrimidinyl, thienyl, thiazolyl, furyl, pyrazolyl, 1,3-oxazolyl or imidazolyl, all of which can be substituted,
- $R^2$ is $(C_1-C_4)$haloalkyl,
- $R^3$ is H or $(C_1-C_4)$alkyl,
- $R^4$ is H, $(C_1-C_4)$alkyl, -CN or -C≡CH,
- A is $CH_2$, O or S,
- B is CH, N or $C(CH_3)$,
- $R^5$ is H or halogen and
- $R^6$ is phenoxy or benzyl, each of which can be monosubstituted to pentasubstituted by halogen or $(C_1-C_3)$haloalkyl.

**2.** A compound of the formulae I and II of claim 1, in which

- $R^1$ is a radical of the formula A, B, C, D, E or F

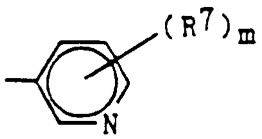

(A),

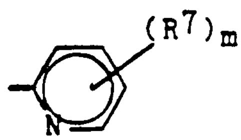

(B),

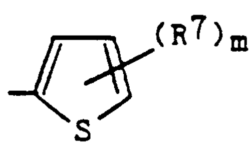

(C),

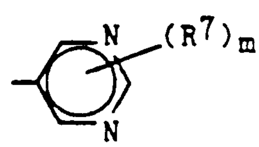

(D),

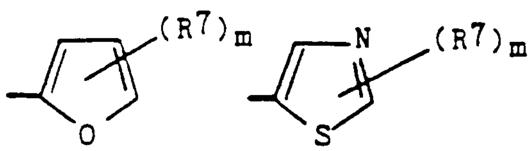

(E), (F)

in which $R^7$ is H, halogen, $(C_1-C_4)$alkyl, $(C_1-C_4)$alkoxy, $(C_1-C_4)$haloalkyl, $(C_1-C_4)$haloalkoxy and m is 1 or 2,

- $R^2$ is $CF_3$,
- $R^3$ is H or methyl,

B is CH,
$R^4$ is H, methyl, -CN or -C≡CH and
$R^6$ is phenoxy which can be substituted in the 4-position by Cl, Br or $CF_3$.

3. A compound of the formulae I and II of claim 2, where $R^7$ is Cl, Br, $(C_1-C_3)$alkoxy, $-OCF_3$, $-OCH_2CF_3$ or $-OCF_2CF_2H$.

4. A compound as claimed in one or more of claims 1 to 3, wherein $R^1$ is a radical of the formula A, B or C.

5. A compound as claimed in one or more of claims 1 to 4, wherein $R^1$ is a radical of the formula A.

6. A process for the preparation of compounds of the formulae I and II as claimed in one or more of claims 1 to 5, which comprises

a) for compounds of the formula I, reacting a compound of the formula III

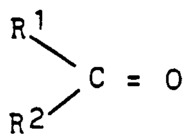

(III)

where $R^1$ and $R^2$ are as defined for formula I, with a compound of the formula IV

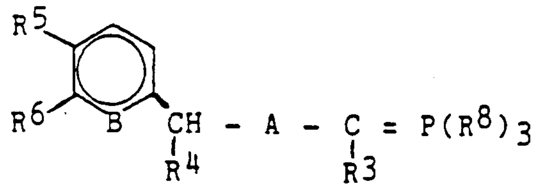

(IV)

where $R^3$, $R^4$, $R^5$, $R^6$, A and B are as defined for formula I and $R^8$ is phenyl, cyclohexyl or butyl, and

b) for compounds of the formula II, catalytically hydrogenating the olefin function of the corresponding compound of the formula I with hydrogen or a hydrogen-liberating compound.

7. An insecticidal or acaricidal agent, containing an effective amount of a compound of the formula I and/or II.

8. The use of the compounds of the formulae I and/or II for controlling insect pests or acarids.

9. A method of controlling insect pests or acarids, which comprises applying an effective amount of a compound of the formula I and/or II to these insect pests or acarids or to the plants, areas or substrates infested with them.

**Claims for the following Contracting State : ES**

1. A process for the preparation of compounds of the formulae I and II, the stereoisomers thereof, and the mixtures of the latter

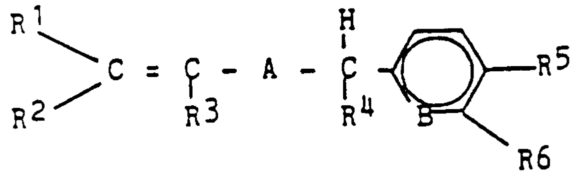

(I)

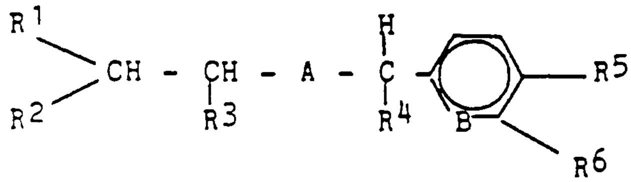

(II)

where

$R^1$ is pyridyl, pyrimidinyl, thienyl, thiazolyl, furyl, pyrazolyl, 1,3-oxazolyl or imidazolyl, all of which can be substituted,

$R^2$ is $(C_1-C_4)$haloalkyl,

$R^3$ is H or $(C_1-C_4)$alkyl,

$R^4$ is H, $(C_1-C_4)$alkyl, -CN or -C≡CH,

A is $CH_2$, O or S,

B is CH, N or $C(CH_3)$,

$R^5$ is H or halogen and

$R^6$ is phenoxy or benzyl, each of which can be monosubstituted to pentasubstituted by halogen or $(C_1-C_3)$haloalkyl, which comprises

a) for compounds of the formula I, reacting a compound of the formula III

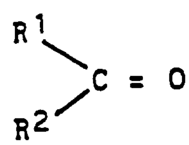

(III)

where $R^1$ and $R^2$ are as defined for formula I, with a compound of the formula IV

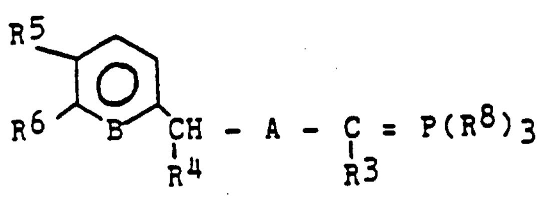

(IV)

where $R^3$, $R^4$, $R^5$, $R^6$, A and B are as defined for formula I and $R^8$ is phenyl, cyclohexyl or butyl, and

b) for compounds of the formula II, catalytically hydrogenating the olefin function of the corresponding compound of the formula I with hydrogen or a hydrogen-liberating compound.

**2.** The process as claimed in claim 1, wherein, in formulae I and II,

$R^1$ is a radical of the formula A, B, C, D, E or F

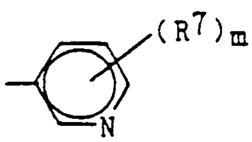
(A),

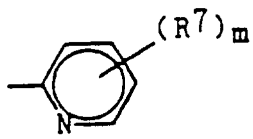
(B),

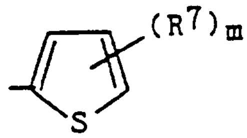
(C),

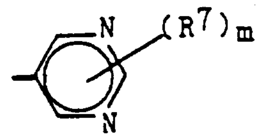
(D),

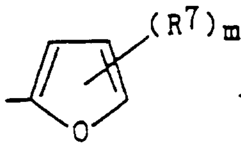
(E),

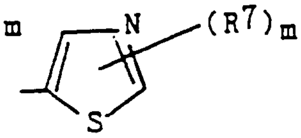
(F)

in which $R^7$ is H, halogen, $(C_1\text{-}C_4)$alkyl, $(C_1\text{-}C_4)$alkoxy, $(C_1\text{-}C_4)$haloalkyl, $(C_1\text{-}C_4)$haloalkoxy and m is 1 or 2,

$R^2$ is $CF_3$,

$R^3$ is H or methyl,

B is CH,

$R^4$ is H, methyl, -CN or -C≡CH and

$R^6$ is phenoxy which can be substituted in the 4-position by Cl, Br or $CF_3$.

**3.** The process as claimed in claim 1 or 2, wherein, in formulae I and II, $R^7$ is Cl, Br, $(C_1\text{-}C_3)$alkoxy, $-OCF_3$, $-OCH_2CF_3$ or $-OCF_2CF_2H$.

**4.** The process as claimed in any one of claims 1 to 3, wherein, in formulae I and II, $R^1$ is a radical of the formula A, B or C.

**5.** The process as claimed in any one of claims 1 to 4, wherein, in formulae I and II, $R^1$ is a radical of the formula A.

**6.** An insecticidal or acaricidal agent, containing an effective amount of a compound of the formula I and/or II as claimed in any one of claims 1 to 5.

**7.** The use of the compounds of the formulae I and/or II as claimed in any one of claims 1 to 5 for controlling insect pests or acarids.

**8.** A method of controlling insect pests or acarids, which comprises applying an effective amount of a compound of the formula I and/or II as claimed in any one of claims 1 to 5 to these insect pests or acarids or to the plants, areas or substrates infested with them.

**Revendications**

**Revendications pour les Etats contractants suivants : AT, BE, CH, DE, FR, GB, IT, LI, NL, SE**

**1.** Composés de formules I et II ci-après, leurs stéréo-isomères et mélanges de ces composés :

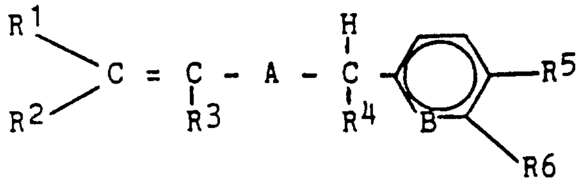

(I),

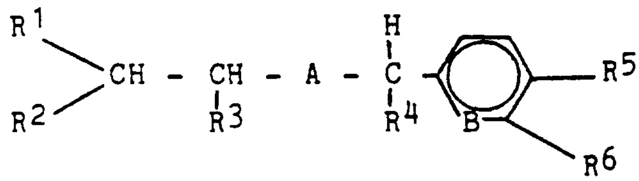

(II),

formules dans lesquelles :

- $R^1$ représente un radical pyridyle, pyrimidyle, thiényle, thiazolyle, furannyle, pyrazolyle, 1,3-oxazolyle ou imidazolyle, qui peuvent tous avoir des substituants,
- $R^2$ un $(C_1\text{-}C_4)$-haloalkyle,
- $R^3$ l'hydrogène ou un $(C_1\text{-}C_4)$-alkyle,
- $R^4$ l'hydrogène, un $(C_1\text{-}C_4)$-alkyle ou un groupe -CN ou -C≡CH,
- A un groupe $CH_2$, O ou S,
- B un groupe CH, N ou $C(CH_3)$,
- $R^5$ l'hydrogène ou un halogène, et
- $R^6$ un groupe phénoxy ou benzyle qui peuvent avoir de 1 à 5 substituants pris parmi les halogènes et des halogénoalkyles en $C_1\text{-}C_3$.

**2.** Composés de formules I et II selon la revendication 1, dans lesquels $R^1$ est un radical de formule A, B, C, D, E ou F :

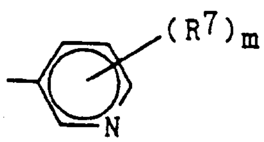

(A),

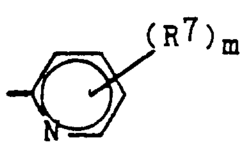

(B),

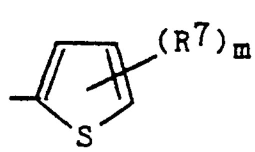

(C),

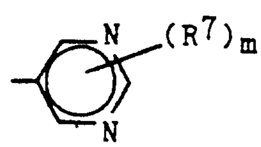

(D),

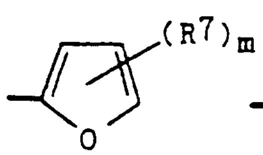

(E),

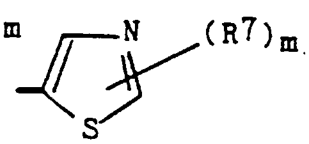

(F)

- $R^7$ désignant l'hydrogène, un halogène, un $(C_1\text{-}C_4)$-alkyle, un $(C_1\text{-}C_4)$-alcoxy, un $(C_1\text{-}C_4)$-haloalkyle ou un $(C_1\text{-}C_4)$-haloalcoxy et m le nombre 1 ou 2,
- $R^2$ est le groupe $CF_3$,

$R^3$ l'hydrogène ou le groupe méthyle,
B le groupe CH,
$R^4$ l'hydrogène ou un groupe méthyle, -CN ou -C≡CH, et
$R^6$ un groupe phénoxy pouvant avoir à la position 4 un atome de chlore ou de brome ou un groupe $CF_3$.

**3.** Composés de formules I et II selon la revendication 2, dans lesquels $R^7$ est le chlore ou le brome ou un groupe $(C_1\text{-}C_3)$-alcoxy, $-OCF_3$, $-OCH_2CF_3$ ou $-OCF_2CF_2H$.

**4.** Composés selon une ou plusieurs des revendications 1 à 3, caractérisés en ce que $R^1$ est un radical de formule A, B ou C.

**5.** Composés selon une ou plusieurs des revendications 1 à 4, caractérisés en ce que $R^1$ est un radical de formule A.

**6.** Procédé de préparation des composés de formules I et II selon une ou plusieurs des revendications 1 à 5, caractérisé en ce que :
a) pour les composés de formule I, on fait réagir un composé de formule III :

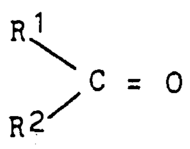

(III),

dans laquelle $R^1$ et $R^2$ ont la même signification que dans la formule I avec un composé de formule IV :

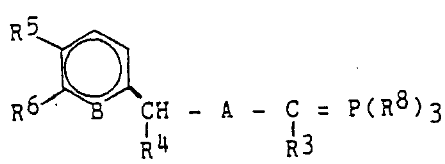

(IV),

dans laquelle $R^3$, $R^4$, $R^5$, $R^6$, A et B ont la même signification que dans la formule I et $R^8$ désignant un groupe phényle, cyclohexyle ou butyle, et
b) pour les composés de formule II, on hydrogène par catalyse la fonction oléfinique des composés correspondants de formule I avec de l'hydrogène ou un composé libérant de l'hydrogène.

**7.** Produits insecticides ou acaricides, caractérisés en ce qu'ils contiennent une proportion efficace d'un ou plusieurs composés des formules I et/ou II.

**8.** Emploi des composés de formules I et/ou II pour lutter contre des insectes ou acariens nuisibles.

**9.** Procédé de lutte contre les insectes ou acariens nuisibles, caractérisé en ce qu'on leur applique, ou bien sur les plantes, surfaces ou substrats qu'ils attaquent, une quantité efficace d'un ou plusieurs composés, des formules I et/ou II précédentes.

**Revendications pour l'Etat contractant suivant : ES**

**1.** Procédé de préparation de composés de formules I et II ci-après, de leurs stéréo-isomères ou de mélanges de ces composés :

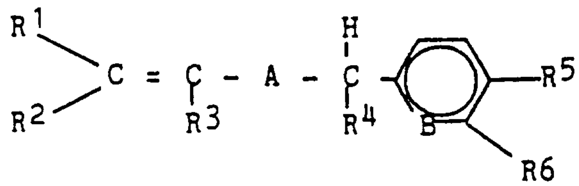

(I),

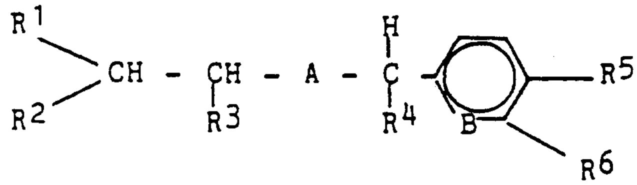

(II'),

dans lesquelles :

$R^1$ représente un radical pyridyle, pyrimidyle, thiényle, thiazolyle, furannyle, pyrazolyle, 1,3-oxazolyle ou imidazolyle, qui peuvent tous avoir des substituants,

$R^2$ un $(C_1-C_4)$-haloalkyle,

$R^3$ l'hydrogène ou un $(C_1-C_4)$-alkyle,

$R^4$ l'hydrogène, un $(C_1-C_4)$-alkyle ou un groupe -CN ou -C≡CH,

A un groupe $CH_2$, O ou S,

B un groupe CH, N ou $C(CH_3)$,

$R^5$ l'hydrogène ou un halogène, et

$R^6$ un groupe phénoxy ou benzyle qui peuvent avoir de 1 à 5 substituants pris parmi les halogènes et des halogénoalkyles en $C_1-C_3$,

procédé caractérisé en ce que :

a) pour les composés de formule I, on fait réagir un composé de formule III :

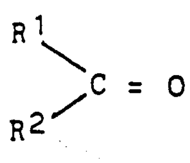

(III),

dans laquelle $R^1$ et $R^2$ ont la même signification que dans la formule I avec un composé de formule IV :

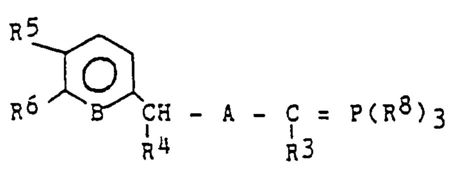

(IV),

dans laquelle $R^3$, $R^4$, $R^5$, $R^6$, A et B ont la même signification que dans la formule I et $R^8$ désignant un groupe phényle, cyclohexyle ou butyle, et

b) pour les composés de formule II, on hydrogène par catalyse la fonction oléfinique des composés correspondants de formule I avec de l'hydrogène ou un composé libérant de l'hydrogène.

**2.** Procédé selon la revendication 1, caractérisé en ce que, dans les formules I et II :

$R^1$ est un radical de formule A, B, C, D, E ou F :

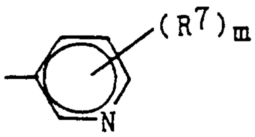
(A),
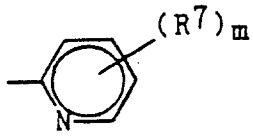
(B),
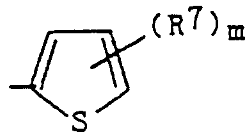
(C),
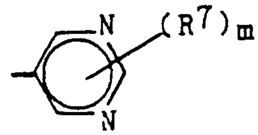
(D),

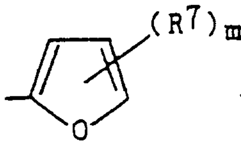
(E),
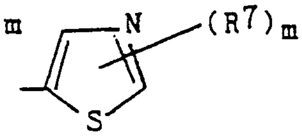
(F)

$R^7$ désignant l'hydrogène, un halogène, un $(C_1\text{-}C_4)$-alkyle, un $(C_1\text{-}C_4)$-alcoxy, un $(C_1\text{-}C_4)$-haloalkyle ou un $(C_1\text{-}C_4)$-haloalcoxy et m le nombre 1 ou 2,
$R^2$ est le groupe $CF_3$,
$R^3$ l'hydrogène ou le groupe méthyle,
B le groupe CH,
$R^4$ l'hydrogène ou un groupe méthyle, -CN ou -C≡CH, et
$R^6$ un groupe phénoxy pouvant avoir à la position 4 un atome de chlore ou de brome ou un groupe $CF_3$.

**3.** Procédé selon la revendication 1 ou 2, caractérisé en ce que, dans les formules I et II, $R^7$ est le chlore ou le brome ou un groupe $(C_1\text{-}C_3)$-alcoxy, $-OCF_3$, $-OCH_2CF_3$ ou $-OCF_2CF_2H$.

**4.** Procédé selon les revendications 1 à 3, caractérisé en ce que, dans les formules I et II, $R^1$ est un radical de formule A, B ou C.

**5.** Procédé selon les revendications 1 à 4, caractérisé en ce que, dans les formules I et II, $R^1$ est un radical de formule A.

**6.** Produits insecticides ou acaricides, caractérisés en ce qu'ils contiennent une proportion efficace d'un composé des formules I et/ou II selon les revendications 1 à 5.

**7.** Emploi des composés de formules I et/ou II selon les revendications 1 à 5, pour lutter contre des insectes ou acariens nuisibles.

**8.** Procédé de lutte contre les insectes ou acariens nuisibles, caractérisé en ce qu'on leur applique, ou bien sur les plantes, surfaces ou substrats qu'ils attaquent, une quantité efficace d'un composé des formules I et/ou II selon les revendications 1 à 5.